# EUROPÄISCHE PATENTSCHRIFT

(11) **EP 0 811 602 B1**
(45) Veröffentlichungstag und Bekanntmachung des Hinweises auf die Patenterteilung: **28.07.1999**
(21) Anmeldenummer: 97105740.1
(22) Anmeldetag: 08.04.1997
(51) Int. Cl.: C07C 227/04

(54) **Verfahren zur Herstellung von Aminomalonester-Salzen**
Process for the preparation of salts of the aminomalonic ester
Procédé de préparation des sels des ester de l'acide aminomalonique

(30) Priorität: 04.06.1996 DE 19622325
(43) Veröffentlichungstag der Anmeldung: 10.12.1997
(73) Patentinhaber: HÜLS AKTIENGESELLSCHAFT, 45764 Marl (DE)
(72) Erfinder: Bauer, Frank, Dr., 51143 Köln (DE); Feld, Marcel, Dr., 51147 Köln (DE)

(56) Entgegenhaltungen:
- "Organic Synthesis, Vol. 40" 1960 , JOHN WILEY & SONS , NEW YORK XP002042883 * Seite 24 - Seite 26 *
- H.R.Christen:Grundlagen der Chemie S.362

## Beschreibung

Die Erfindung betrifft ein verbessertes Verfahren zur Herstellung von Aminomalonsäureestern durch katalytische Hydrierung der entsprechenden Nitro-. Nitroso- oder gegebenenfalls 0-substituierten Hydroxyiminoverbindungen. Die Aminomalonsäureester können durch Neutralisation mit einer Säure in das Aminomalonsäureester-Salz überführt werden.

Aminomalonsäureester sowie deren Salze mit Ammoniumstruktur sind wertvolle Bausteine für die Synthese von Heterocyclen. Eine Reihe von Herstellverfahren für Aminomalonester ist bekanntgeworden. bei denen die entsprechenden Nitro-, Nitroso- oder gegebenenfalls 0-substituierten Hydroxyiminoverbindungen reduziert werden. So beschreiben Gabriel et al. (B. **27** [1894]. 1141) die Reduktion mittels Zinn. Zinn(II)-chlorid und Salzsäure. Nach Cherchez (Bull.Soc.Chim.Fr., (4) **47** [1930]. 1282) sowie nach Lindemann et al. (Chem.Ber.. **63** [1930]. 710) kann man amalgamiertes Aluminium als Reduktionsmittel verwenden. Siya et al. (Syntheses, **1986**, 133) reduzieren die Ausgangsstoffe mit Ammoniumformiat. Nach FR-1.171.906 kann man die Aminomalonsäureester auch elektrochemisch durch kathodische Reduktion herstellen.

Diese Verfahren arbeiten überwiegend mit teuren Reduktionsmitteln. Zudem fallen dabei große Mengen an Salzen an, die schwierig zu entsorgen sind. Das elektrochemische Verfahren eignet sich im technischen Maßstab nur für Hersteller, die auf Elektrochemie eingerichtet sind.

Wesseley et al. (Monatsh.Chem., **83** [1952]. 678.687) beschreiben die Reduktion der genannten Ausgangsstoffe durch katalytische Hydrierung mit Katalysatoren, wie Raney-Nickel. Palladium auf Aktivkohle und Platin auf Aktivkohle. Eine selektive Hydrierung unter Erhalt der Carbonestergruppen gelingt. weil deren Umwandlung in Methylolgruppen viel energischere Bedingungen erfordert als die Hydrierung der Nitro-, Nitroso- oder Hydroxyiminogruppen zu Aminogruppen. Solche Bedingungen, d.h. beispielsweise Temperaturen deutlich oberhalb von 100°C, sind schon deshalb nicht empfehlenswert. weil Aminomalonester empfindliche Moleküle sind. die zu intermolekularen Kondensationsreaktionen neigen. Aus diesem Grunde ist es auch ratsam. die Aminomalonester in Form ihrer Salze zu gewinnen, wenn man sie nicht gleich im Reaktionsgemisch weiter umsetzen will.

Die katalytische Hydrierung der Nitro-. Nitroso- und Hydroxyiminomalonester wurde stets in einem inerten Lösungsmittel durchgeführt. Erwünscht ist ein Lösungsmittel, in dem das Aminomalonester-Salz unlöslich oder möglichst wenig löslich ist. Setzt man jedoch ein solches Lösungsmittel ein. z.B. Toluol. Methylacetat, Ethylacetat oder Methyl-tert.butylether, so verläuft die Hydrierung sehr langsam und unvollständig. Dem kann man zwar entgegenwirken, indem man mit großer Verdünnung arbeitet. Das führt jedoch zu Raum-Zeit-Ausbeuten, die für den technischen Maßstab nicht akzeptabel sind.

Sowohl befriedigende Reaktionsgeschwindigkeiten als auch gute Raum-Zeit-Ausbeuten lassen sich mit Ethanol als Lösungsmittel erzielen. Darin sind aber die Aminomalonester-Salze gut löslich, so daß sie nicht durch Fällung abgetrennt werden können. Einen Ausweg aus dem Dilemma. daß manche Lösungsmittel für die Hydrierstufe gut, aber für die Abtrennung des Salzes wenig geeignet sind, und andere Lösungsmittel sich umgekehrt verhalten, strebt das Verfahren nach Org. Synth., **40**, 24 an. Danach wird die Hydrierung in Ethanol durchgeführt, das Lösungsmittel bei maximal 50°C abdestilliert, der Rückstand in Diethylether aufgenommen und das Aminomalonester-Hydrochlorid durch Einleiten von Chlorwasserstoffgas ausgefällt. Dieses Verfahren ist jedoch aufwendiger als die erwünschte Arbeitsweise mit demselben Lösungsmittel in der Hydrierungs- und in der Fällungsstufe. Außerdem führt es besonders im technischen Maßstab zu erhöhten Produktverlusten infolge der erwähnten Neigung der Aminomalonester zu Kondensationsreaktionen. Diese Neigung ist natürlich, der Umkehr des Verdünnungsprinzips folgend, in dem Maße stärker ausgeprägt, in dem das Ethanol abdestilliert und die Konzentration des Aminomalonesters ansteigt, bis er schließlich in Substanz vorliegt.

Diese Produktverluste durch Kondensationsreaktionen werden zwar weitgehend vermieden, wenn man zunächst die Hydrierung in Ethanol ausführt und dann dem Hydriergemisch vor der Salzfällung ein unpolares Lösungsmittel, wie Toluol, zusetzt. Die Ausbeute an Aminomalonester-Salz ist dann jedoch unbefriedigend, da dieses in dem Lösungsmittelgemisch recht gut löslich ist. Zudem ist dieses Lösungsmittelgemisch nach dem Abfiltrieren des Salzes ohne Aufarbeitung nicht wiederverwendbar.

Es wurde nun überraschend gefunden, daß sich Aminomalonester der allgemeinen Formel I : 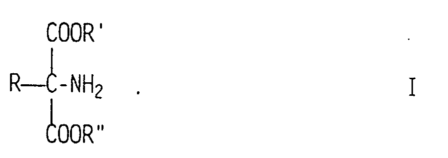 in der R' und R" gleich oder verschieden sein können und Alkyl-, Cycloalkyl-, Aryl-. Alkaryl- oder Aralkylreste bedeuten. R dieselbe Bedeutung hat wie R' und R" und zusätzlich für Wasserstoff stehen kann,
sowie gegebenenfalls deren Salze der allgemeinen Formel II: 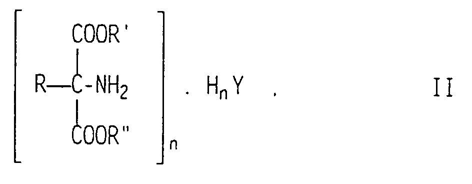 in der R', R" und R die für die Formel I angegebene Bedeutung haben, Y ein Säureanion bezeichnet und n die Basizität der Säure HₙY bedeutet,
durch katalytische Hydrierung eines substituierten Malonesters der allgemeinen Formel III: 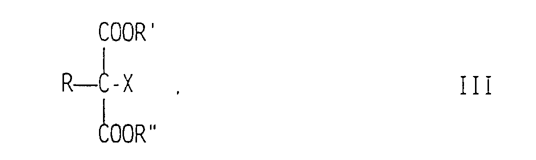 in der R', R" und R die für die Formel I angegebene Bedeutung haben und X eine Nitrogruppe, Nitrosogruppe oder, wenn R für Wasserstoff steht, eine durch Umlagerung aus der Nitrosogruppe erhältliche Hydroxyiminogruppe, die O-substituiert sein kann, bezeichnet,
in einem inerten Lösungsmittel und gegebenenfalls durch Zusatz einer Säure der Formel HₙY, in der Y und n die für die Formel II angegebene Bedeutung haben, vorteilhaft herstellen lassen, wenn man die Hydrierung in Gegenwart eines festen Trockenmittels sowie eines Lösungsmittels oder Lösungsmittelgemisches durchführt, in dem das Aminomalonester-Salz II weitgehend unlöslich ist.

Das Verfahren nach der Erfindung liefert in der Hydrierstufe die Aminomalonester I mit hohen Ausbeuten in einer rasch ablaufenden Reaktion und daher mit hohen Raum-Zeit-Ausbeuten, auch bei Verwendung von Lösungsmitteln, die im Unterschied zu Ethanol keine Hydroxylgruppen enthalten. Letzteres ist von Vorteil, wenn weitere Umsetzungen des Aminomalonesters I gleich im Reaktionsgemisch mit Partnern geplant sind, die alkoholyseempfindliche Substituenten enthalten. In der Neutralisationsstufe werden die Aminomalonester-Salze II in hoher Reinheit und, bezogen auf die Aminomalonester I aus der Hydrierstufe, in praktisch quantitativer Ausbeute gewonnen. In beiden Stufen des Verfahrens werden dieselben Lösungsmittel eingesetzt. Das können Lösungsmittelgemische sein. die dann jedoch nicht aufgearbeitet werden müssen. sondern ebenso wie die einheitlichen Lösungsmittel als solche für einen neuen Ansatz verwendet werden können.

In den als Ausgangsstoffe bevorzugten substituierten Malonestern III, und dementsprechend auch in den bevorzugten Aminomalonestern I und deren Salzen II, bedeuten R' und R" gleiche Alkylreste mit 1 bis 4 Kohlenstoffatomen und steht R vorzugsweise für Wasserstoff oder einen Kohlenwasserstoffrest mit 1 bis 8 Kohlenstoffatomen. insbesondere einen Alkylrest mit 1 bis 4 Kohlenstoffatomen. In den bevorzugten substituierten Malonestern III bedeutet X die Nitrogruppe oder, wenn R für Wasserstoff steht, die durch Umlagerung entstandene Hydroxyiminogruppe, die durch einen Kohlenwasserstoffrest mit 1 bis 8 Kohlenstoffatomen, insbesondere einen Alkylrest mit 1 bis 4 Kohlenstoffatomen, oder einen Acylrest mit 2 bis 8 Kohlenstoffatomen O-substituiert sein kann. Die substituierten Malonester III können einzeln oder im Gemisch eingesetzt werden.

Geeignete substituierte Malonester III sind z.B. Nitromalonsäuredimethylester, Nitromalonsäurediethylester, Nitromalonsäuredibenzylester, Methylnitromalonsäuredimethylester, Methylnitrosomalonsäuredimethylester. Isobutylnitrosomalonsäurediethylester, Hydroxyiminomalonsäurediethylester. O-Acetylhydroxyiminomalonsäuredimethylester, O-Benzoylhydroxyiminomalonsäurediethylester, O-Methylhydroxyiminomalonsäurediethylester, O-Phenylhydroxyiminomalonsäurediisopropylester, O-n-Butylhydroxyiminomalonsäuredi-n-butylester, O-n-Octylhydroxyiminomalonsäuredimethylester. Methylnitromalonsäurediethylester, O-Acetylhydroxyiminomalonsäurediethylester, Hydroxyiminomalonsäuredimethylester, Hydoxyiminomalonsäurediisopropylester usw.

Die erste Stufe des Verfahrens ist eine katalytische Hydrierung. Dabei werden die Katalysatoren verwendet, die sich bekanntermaßen für die Hydrierung von Nitro-, Nitroso- und gegebenenfalls 0-substituierten Hydroxyiminogruppen zu Aminogruppen eignen, wie Platin-, Palladium-, Rhodium und Nickelkatalysatoren. Die katalytisch wirksamen Metalle können isoliert vorliegen oder auf einem Träger aufgebracht sein. Als Beispiele seien Raney-Nickel, Palladiummohr, Palladium auf Aktivkohle. Platinmohr, Platin auf Aktivkohle, Palladium/Platin auf Aluminiumoxid. Siliciumdioxid. Calciumcarbonat oder Bariumsulfat sowie Platin- durch orientierende Versuche unschwer ermittelt werden. Edelmetallkatalysatoren werden im allgemeinen in Mengen von 0,0001 bis 0,05 Gewichtsteilen- Metall pro Gewichtsteil substituierten Malonsäureester III eingesetzt.

Ein wichtiges Merkmal der Erfindung ist die Mitverwendung eines festen Trockenmittels in der Hydrierstufe. Dafür eignen sich alle bekannten festen Trockenmittel, die das bei der Hydrierungsreaktion entstehende Wasser bei der jeweiligen Reaktionstemperatur. z.B. als Kristallwasser, zu binden vermögen. Geeignete feste Trockenmittel sind überwiegend anorganischer Natur und arbeiten reversibel, können also durch Erhitzen regeneriert werden, nachdem sie mit Wasser vollständig beladen sind. Beispiele für geeignete Trockenmittel sind Calciumchlorid. Natriumsulfat, Magnesiumsulfat, Natriumacetat, Natriumcarbonat. Kaliumcarbonat, Calciumsulfat, Aluminiumoxid, Kieselgele, trockene Kunstharzionenaustauscher, Kupfer(II)-sulfat, Monmorillonite, Calciumoxid, Bariumoxid und Magnesiumoxid. Auch die als Molekularsiebe bekannten natürlichen oder synthetischen Zeolithe eignen sich hervorragend für das Verfahren nach der Erfindung.

Die Menge des festen Trockenmittels muß so gewählt werden. daß sie die Abscheidung einer wässerigen Phase im Reaktionsgemisch verhindert. Die Mindestmenge hängt von dem jeweiligen substituierten Malonester III (z.B. ergeben Nitroverbindungen doppelt so viel Wasser wie Hydroxyiminoverbindungen). dem Wasseraufnahmevermögen des gewählten Trockenmittels und dem Wasserlösevermögen des Lösungsmittels oder Lösungsmittelgemisches bei der Reaktionstemperatur ab. Es können aber auch ohne weiteres größere Mengen an Trockenmittel verwendet werden. Bei einer absatzweisen Arbeitsweise ist eine Limitierung nur dadurch gegeben. daß das Hydrierungsgemisch durchmischbar bleiben muß. Exzessive Mengen Trockenmittel erschweren allerdings die Aufarbeitung, die eine Fest-Flüssig-Trennung erfordert. Die Hydrierungsgemische werden in der Regel schwierig handhabbar, wenn ihr Gehalt an an festem Trockenmittel 50 Gewichtsprozent überschreitet. In der Praxis hat sich gezeigt, daß man zweckmäßig mindestens 0,02 Gewichtsteile festes Trockenmittel pro Gewichtsteil substituierten Malonester III verwenden sollte. Vorteilhaft wendet man das feste Trockenmittel in Mengen von 0,02 bis 4.0 Gewichtsteilen, insbesondere von 0,05 bis 0,25 Gewichtsteilen pro Gewichtsteil substituierter Malonester III an. Die optimale Menge an festem Trockenmittel läßt sich durch orientierende Versuche leicht ermitteln.

Bei einer kontinuierlichen Arbeitsweise mit in Säulenform fest angeordnetem Hydrierkatalysator und festem Trockenmittel, wie in der Folge beschrieben, wird das Trockenmittel fortschreitend mit Wasser beladen, und die Erschöpfung des Trockenmittels wird angezeigt, wenn sich im Durchlauf Wasser abscheidet und nicht umgesetzter substituierter Malonester III nachgewiesen werden kann. Auf diese Weise wird das Wasseraufnahmevermögen des festen Trockenmittels optimal genutzt.

Ein weiteres wichtiges Merkmal des Verfahrens nach der Erfindung ist die Verwendung eines Lösungsmittels oder Lösungsmittelgemisches, in dem das Aminomalonester-Salz II weitgehend unlöslich ist. Diese Bedingung erfüllen unpolare oder schwach polare organische Lösungsmittel die mit Wasser praktisch nicht oder nur in begrenztem Umfang mischbar sind. Dazu zählen Carbonsäureester, insbesondere solche von C₂-C₄-Carbonsäuren mit C₁-C₄-Alkanolen, bei der Reaktionstemperatur flüssige aliphatische und aromatische Kohlenwasserstoffe, halogenierte und insbesondere chlorierte aliphatische oder aromatische Kohlenwasserstoffe, einwertige Alkohole mit 4 bis 12 Kohlenstoffatomen, aromatische und aliphatische Mononitrile mit 2 bis 13 Kohlenstoffatomen und Dialkylether mit 4 bis 8 Kohlenstoffatomen. Geeignete Lösungsmittel dieser Art, die für sich allein oder im Gemisch miteinander eingesetzt werden können. sind z.B., n-Heptan und n-Octan sowie deren Isomere, Benzol, Toluol, Ethylbenzol, 1,2-Dichlorethan, n-Octanol, n-Dodekanol, 2-Ethyl-1-hexanol, Benzonitril, Caprylnitril, Xylol. Diethylether, Diisopropylether. Methyl-tert.butylether und Acetonitril. Besonders bevorzugte Lösungsmittel sind Methylacetat und Ethylacetat.

In der Praxis hat es sich als nützlich erwiesen, wenn das Lösungsmittel oder Lösungsmittelgemisch eine kleine Menge Wasser. d.h. bei Raumtemperatur bis zu etwa 20 Gewichtsprozent. zu lösen vermag. Wenn man ein Lösungsmittel, wie n-Heptan oder Toluol gewählt hat. das praktisch kein Wasser löst, kann man eine damit mischbare hydrophile Verbindung in einer solchen Menge zusetzen, daß die Löslichkeit von Wasser in dem Gemisch in dem genannten Bereich liegt. Lösungsmittel oder Lösungsmittelgemische mit größerem Lösevermögen für Wasser sollten zumindest dann vermieden werden, wenn das Aminomalonester-Salz ausgefällt werden soll, weil sonst dessen Löslichkeit merklich zunimmt.

Das Verfahren nach der Erfindung kann kontinuierlich oder absatzweise durchgeführt werden. Bei einer absatzweisen Arbeitsweise kann man die Lösung des substituierten Malonesters III, den Hydrierkatalysator und das Trockenmittel in einem Druckreaktor vorlegen, das Gemisch auf die Reaktionstemperatur erhitzen und solange Wasserstoff aufpressen, bis der Druck konstant bleibt. Dabei sorgt man zweckmäßig für gute Durchmischung des Reaktionsgemisches. Um eine annehmbare Reaktionsgeschwindigkeit zu erreichen, arbeitet man im allgemeinen, je nach Katalysator, bei 0 bis 100 °C und unter einem Wasserstoffdruck von 1 bis 150 bar (abs.). Bevorzugte Hydriertemperaturen sind 0 bis 50°C, insbesondere 20 bis 40°C. Aus dem Reaktionsgemisch werden die festen Anteile, d.h. Katalysator und Trockenmittel, durch eine übliche Fest-Flüssig-Trennung, z.B. mittels Schwerkraftfiltration oder durch Absaugen, von der flüssigen Phase getrennt. Durch Waschen der abgetrennten festen Anteile mit dem verwendeten Lösungsmittel wird die Ausbeute verbessert. Der Aminomalonester ist in der flüssigen Phase gelöst und kann darin gewünschtenfalls weiter umgesetzt oder als Salz ausgefällt werden. Dazu kann man die Säure HₙY in die im Interesse einer möglichst vollständigen Fällung zweckmäßig auf Raumtemperatur abgekühlte Lösung einbringen und das ausgefällte Aminomalonester-Salz II. wiederum in üblicher Weise. von dem Lösungsmittel abtrennen. Das Lösungsmittel kann ohne weitere Behandlung für einen neuen Ansatz verwendet werden. Dies kann mehrere Male wiederholt werden, bis sich Verunreinigungen, z.B. Nebenprodukte, so stark anreichern, daß die Ausbeute und/oder die Reinheit des Aminomalonester-Salzes II beeinträchtigt werden. In diesem Fall kann das Lösungsmittel(gemisch) durch Destillation aufgearbeitet werden.

Die Säure HₙY kann eine ein- oder mehrbasische, vorteilhaft bis zu dreibasische organische oder anorganische Säure sein. Geeignete Säuren sind u.a. Chlorwasserstoff, Bromwasserstoff, Schwefelsäure. Phosphorsäure, Essigsäure, Oxalsäure, Methansulfonsäure und p-Toluolsulfonsäure. Die Säuren können der Lösung gegebenenfalls gasförmig, in unverdünnter flüssiger oder fester Form oder in einem Lösungsmittel - zweckmäßig dem Lösungsmittel der Hydrierstufe - gelöst zugeführt werden.

Das Trockenmittel, das im Gemisch mit dem Hydrierkatalysator vorliegt. kann in üblicher Weise regeneriert. das heißt vom aufgenommenen Wasser befreit werden. Dazu kann man es z.B. durch Erhitzen in einem Inertgasstrom auf 100 bis 300°C, vom aufgenommenen Wasser befreit werden. worauf das Gemisch für einen neuen Ansatz wiederverwendet werden kann. Die optimalen Entwässerungstemperaturen hängen von der Temperatur ab. bei der die Trockenmittel Wasser abzugeben beginnen. So genügen z.B. bei Salz-Hydraten meist niedrigere Temperaturen. während z.B. Zeolithe im oberen Bereich der genannten Temperaturspanne entwässert werden müssen. Das Erhitzen kann, je nach dem Katalysator, im Luftstrom oder in einem Inergasstrom (z.B. von Stickstoff oder Argon, geschehen. Bei einer anderen, weniger bevorzugten Variante kann man das Trockenmittel, sofern es wasserlöslich ist, in Wasser lösen, die Lösung verwerfen und zumindest den verbliebenen Hydrierkatalysator erneut verwenden. Das Verfahren nach der Erfindung kann auch kontinuierlich durchgeführt werden. Dazu kann man den Katalysator und das Trockenmittel. gemischt oder schichtweise, in einem Rohrreaktor fest anordnen. auf die Reaktionstemperaratur erhitzen und dann die Lösung des substituierten Malonesters III über den Katalysator rieseln lassen. während Wasserstoff im Gleich- oder im Gegenstrom dazu geführt wird. Hinsichtlich der Temperatur- und Druckbedingungen unterscheidet sich die kontinuierliche Arbeitsweise nicht von dem zuvor beschriebenen absatzweisen Verfahren. Wenn das Wasseraufnahmevermögen des Trockenmittels erschöpft ist, erscheinen Wasser und nicht umgesetzter substituierter Malonester III im Durchlauf. Das Trockenmittel muß dann regeneriert werden. Dazu kann man Katalysator und Trockenmittel mit dem Lösungsmittel spülen, das verbliebene Lösungsmittel verdampfen, indem man warme Luft durch den Rohrreaktor bläst und schließlich das Wasser aus dem Trockenmittel austreibt, indem man die Temperatur entsprechend steigert und, je nach dem Katalysator, einen Luft- oder Inertgasstrom durch den Rohrreaktor leitet. Bei einer weiteren Ausgestaltung dieses kontinuierlichen Verfahrens sind zwei Rohrreaktoren parallel angeordnet, von denen sich der eine in der Hydrierphase befindet, während in dem anderen das Trockenmittel regeneriert wird.

Wenn bei dem kontinuierlichen Verfahren nicht umgesetzter Malonester III "durchgebrochen" ist oder bei dem diskontinuierlichen Verfahren aus irgendeinem Grunde die Hydrierung unvollständig war, empfiehlt es sich. den Aminomalonester I mit äquimolaren Mengen Säure oder einem Säureunterschuss zu fällen und nach Abtrennung des Aminomalonester-Salzes II das Filtrat in die Hydrierstufe zurückzuführen. Man vermeidet auf diese Weise eine Nachfällung von Aminomalonester-Salz II während der Hydrierung, die den Katalysator inaktivieren könnte.

Die folgenden Beispiele werden gegeben, um die Erfindung weiter zu erläutern, und sollen nicht den Schutzbereich begrenzen, wie er in den Patentansprüchen definiert ist.

### Beispiele.

### Beispiel 1 - Aminomalonsäurediethylester-Hydrochlorid

Eine Lösung von 420,0 g eines Gemisches aus Hydroxyiminomalonsäurediethylester und Acetoxyiminomalonsäurediethylester (zusammen 2.0 Mol) in 400 ml Ethylacetat sowie 40,0 g wasserfreies Magnesiumsulfat und 12,5 g Palladium auf Aktivkohle (5 Gewichtsprozent Pd) als Hydrierkatalysator wurden in einem 1.5 l-Druckreaktor mit Rührer vorgelegt. Bei 30 bis 35°C wurde Wasserstoff mit 20 bar aufgepreßt, bis der Druck nach 80 Minuten konstant blieb. Dabei wurde der Inhalt des Reaktors durch Rühren gut durchmischt. Das Hydriergemisch wurde auf 20°C abgekühlt, mit 200 ml Ethylacetat verdünnt, und das Gemisch aus wasserhaltigem Magnesiumsulfat und Katalysator wurde abfiltriert. Der abgetrennte Feststoff wurde dreimal mit insgesamt 200 ml Ethylacetat gewaschen und die Waschflüssigkeit mit dem Filtrat vereinigt. Durch Einleiten von 68 g trockenem Chlorwasserstoff wurde das Aminomalonsäurediethylester-Hydrochlorid als weißer Feststoff ausgefällt, abfiltriert, mit Ethylacetat gewaschen und im Wasserstrahlvakuum bei 70 °C getrocknet. Die Ausbeute betrug 359.6 g (85.1 % der Theorie), der Schmelzpunkt 165°C und die Reinheit nach dem analytisch bestimmten Chlorgehalt 100%.

### Beispiel 2 - Aminomalonsäurediethylester-Hydrochlorid

Es wurde verfahren wie im Beispiel 1. jedoch wurde das in diesem Beispiel angefallene feste Gemisch aus wasserhaltigem Magnesiumsulfat und Katalysator mit heißem Wasser gewaschen und der nach Trocknung zurückgewonnene Katalysator erneut eingesetzt. Die Ausbeute betrug 358,9 g (84,9 % der Theorie). der Schmelzpunkt 165°C und die Reinheit (nach dem Chlorgehalt) 100 %.

### Beispiel 3 - Aminomalonsäurediethylester-Hydrochlorid

Es wurde verfahren wie im Beispiel 1, jedoch wurde der Ausgangsstoff in einem Gemisch aus 400 ml Ethylacetat und 20 ml Ethanol gelöst und die Magnesiumsulfatmenge auf 10,0 g vermindert. Die Ausbeute betrug 340.2 g (80.5 % der Theorie). der Schmelzpunkt 165 °C und die Reinheit (nach dem Chlorgehalt) 100 %.

### Beispiel 4 Aminomalonsäurediethylester-Hydrochlorid

Es wurde .verfahren wie im Beispiel 1. jedoch wurde als Lösungsmittel Methyl-tert.butylether verwendet. Die Ausbeute betrug 353,1 g (83.6 % d.Th.), der Schmelzpunkt 165 °C und die Ausbeute (nach dem Chlorgehalt) 100 %.

### Beispiel 5 - Aminomalonsäuredibutylester-Hydrochlorid

Es wurde verfahren wie im Beispiel 1, jedoch wurden 423 g eines Gemisches aus Hydroxyiminomalonsäurediisobutylester und Acetoxyiminodiisobutylester (zusammen 1,68 Mol) in Gegenwart von 400 ml Ethylacetat und 40,0 g wasserfreiem Magnesiumsulfat hydriert. Das Aminomalonsäurediisobutylester-Hydrochlorid wurde nach Abtrennung von Magnesiumsulfat und Katalysator durch Einleiten von 64,5 g trockenem Chlorwasserstoff gefällt. Die Ausbeute betrug 339,4 g (75,5 % der Theorie), der Schmelzbereich 93,5 - 95,5 °C. die Reinheit (nach dem Chloridgehalt) 99%.

### Beispiel 6 - Aminomalonsäurediisobutylester-p-Toluolsulfonat

Es wurde verfahren wie in Beispiel 5, jedoch wurden dem Hydriergemisch nach Abtrennung von Magnesiumsulfat und Katalysator 316,9 g p-Toluolsulfonsäure in 2.688 g Ethylacetat zugesetzt, und das Gemisch wurde über Nacht bei 5°C gelagert. Die Ausbeute betrug 47,6 g (61.8 % der Theorie), der Schmelzbereich 131-132 °C.

### Beispiel 7 - Amino-methyl-malonsäurediethylester-p-Toluolsulfonat

Eine Lösung von 250 g Nitro-methyl-malonsäurediethylester in 450 g Ethylacetat sowie 50,0 g wasserfreies Magnesiumsulfat und 15 g Palladium auf Aktivkohle (5 Gewichtsprozent Pd) als Hydrierkatalysator wurden in einem 1.5 l-Druckreaktor mit Rührer vorgelegt. Bei 35 bis 40°C wurde Wasserstoff mit 6 bis 10 bar aufgepreßt, bis der Druck nach 50 Minuten konstant blieb. Dabei wurde der Inhalt des Reaktors durch Rühren gut durchmischt. Das Hydriergemisch wurde auf 20°C abgekühlt und nach Abfiltrieren von Magnesiumsulfat und Katalysator mit einer Lösung von 216.8 g p-Toluolsulfonsäure in 900 g Ethylacetat versetzt. Die Lösung wurde im Wasserstrahlvakuum eingedampft, wobei 383 g (93 % der Theorie) eines hellgelben Öls zurückblieben, das nach ¹³C-NHR-Analyse neben p-Toluolsulfosäure und geringen Mengen weiterer Verunreinigungen hauptsächlich aus Amino-methyl-malonsäurediethylester-p-Toluolsulfonat bestand.

## Patentansprüche

1. Verfahren zur Herstellung von Aminomalonestern der allgemeinen Formel I: 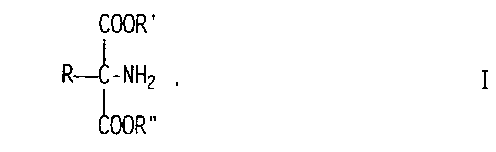 in der R' und R" gleich oder verschieden sein können und Alkyl-, Cycloalkyl-, Aryl-, Alkaryl- oder Aralkylreste bedeuten, R dieselbe Bedeutung hat wie R' und R" und zusätzlich für Wasserstoff stehen kann,
sowie gegebenenfalls deren Salzen der allgemeinen Formel II: 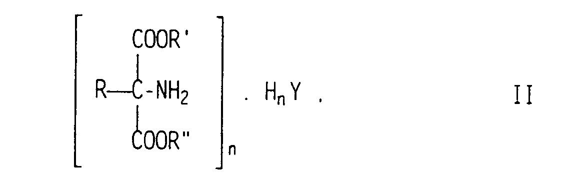 in der R', R" und R die für die Formel I angegebene Bedeutung haben, Y ein Säureanion bezeichnet und n die Basizität der Säure HₙY bedeutet,
durch katalytische Hydrierung eines substituierten Malonesters der allgemeinen Formel III: 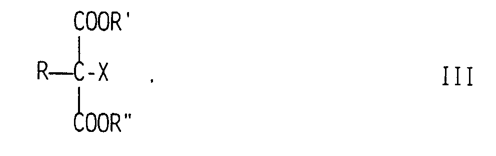 in der R', R", und R die angegebene Bedeutung haben und X eine Nitrogruppe, Nitrosogruppe oder, wenn R für Wasserstoff steht, eine durch Umlagerung aus der Nitrosogruppe erhältliche Hydroxyiminogruppe, die O-substituiert sein kann, bezeichnet,
in einem inerten Lösungsmittel und gegebenenfalls durch Zusatz einer Säure HₙY zu dem Hydrierungsgemisch, dadurch gekennzeichnet, daß man die Hydrierung in Gegenwart eines festen Trockenmittels sowie eines Lösungsmittels oder Lösungsmittelgemisches durchführt, in dem das Aminomalonester-Salz II weitgehend unlöslich ist.

2. Verfahren nach Anspruch 1, **dadurch gekennzeichnet,** daß man als Trockenmittel Calciumchlorid. Natriumsulfat, Magnesiumsulfat. Natriumacetat, Natriumcarbonat, Kaliumcarbonat, Aluminiumoxid, ein Kieselgel. Calciumsulfat, einen trockenen Kunstharzionenaustauscher, ein Molekularsieb oder ein Gemisch dieser Stoffe in einer Menge von 0.02 bis 4,0 Gewichtsteilen pro Gewichtsteil substituierten Malonester III verwendet.

3. Verfahren nach Anspruch 2, **dadurch gekennzeichnet,** daß man das Trockenmittel in einer Menge von 0.05 bis 0,25 Gewichtsteilen pro Gewichtsteil substituierten Malonester III verwendet.

4. Verfahren nach einem der Ansprüche 1 bis 3, **dadurch gekennzeichnet,** daß man als Lösungsmittel ein Alkylacetat, einen gegebenenfalls halogenierten aliphatischen oder aromatischen Kohlenwasserstoff, einen langkettigen aliphatischen Alkohol, ein langkettiges aliphatisches Nitril, einen wasserunlöslichen Ether oder ein Gemisch dieser Lösungsmittel verwendet.

5. Verfahren nach einem der Ansprüche 1 bis 4, **dadurch gekennzeichnet,** daß man absatzweise arbeitet und nach der Hydrierung das Trockenmittel zusammen mit dem Hydrierkatalysator durch eine übliche Fest-Flüssig-Trennung abtrennt.

6. Verfahren nach Anspruch 5, **dadurch gekennzeichnet,** daß das Trockenmittel in Gegenwart des zusammen mit ihm abgetrennten Hydrierkatalysators von Wasser befreit und dann zusammen mit dem Hydrierkatalysator für einen neuen Ansatz verwendet wird.

7. Verfahren nach Anspruch 5, **dadurch gekennzeichnet**, daß das zusammen mit dem Hydrierkatalysator abgetrennte Trockenmittel in Wasser gelöst und der verbliebene Hydrierkatalysator für einen neuen Ansatz verwendet wird.

8. Verfahren nach einem der Ansprüche 1 bis 4, **dadurch gekennzeichnet,** daß die Hydrierung kontinuierlich an einem gegebenenfalls schichtweise aufgebauten Festbett aus Katalysator und Trockenmittel durchgeführt wird, über die der substituierte Malonester III rieselt, während der Wasserstoff im Gleich- oder im Gegenstrom geführt wird.

9. Verfahren nach Anspruch 8, **dadurch gekennzeichnet,** daß das Trockenmittel in dem Festbett. gegebenenfalls nach Waschen des Festbetts mit einem Lösungsmittel zur Entfernung von organischen Verunreinigungen, von aufgenommenem Wasser befreit und das Festbett erneut für die Hydrierung eingesetzt wird.

## Claims

1. A process for the preparation of an aminomalonic ester of the general formula I: 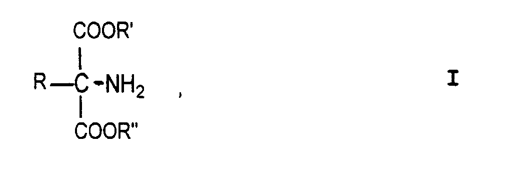 in which R' and R" can be identical or different and are alkyl, cycloalkyl, aryl, alkaryl or aralkyl radicals and R is defined in the same way as R' and R" and can additionally be hydrogen,
or of a salt thereof of the general formula II: 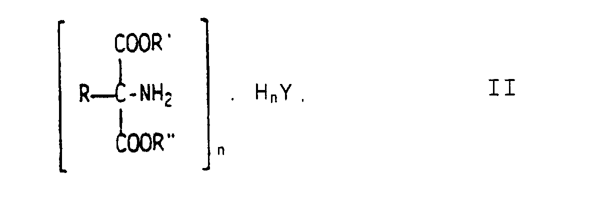 in which R', R" and R are as defined for the formula I, Y is an acid anion and n is the basicity of the acid HₙY,
by catalytic hydrogenation of a substituted malonic ester of the general formula III: 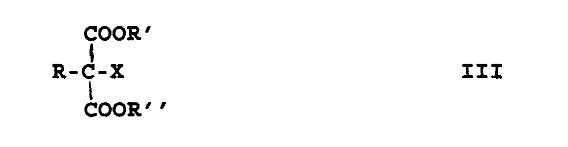 in which R', R" and R are as defined and X is a nitro or nitroso group or, if R is hydrogen, is a hydroxyimino group which is obtainable from the nitroso group by rearrangement and which may be O-substituted,
in an inert solvent and if desired by addition of an acid HₙY to the hydrogenation mixture, characterized in that the hydrogenation is carried out in the presence of a solid dehydrating agent and a solvent or solvent mixture in which the aminomalonic ester salt II is largely insoluble.

2. A process according to claim 1, characterized in that calcium chloride, sodium sulphate, magnesium sulphate, sodium acetate, sodium carbonate, potassium carbonate, aluminium oxide, a silica gel, calcium sulphate, a dry synthetic resin ion exchanger, a molecular sieve or a mixture of these substances is used as dehydrating agent in an amount from 0.02 to 4.0 parts by weight per part by weight of substituted malonic ester III.

3. A process according to claim 2, characterized in that the dehydrating agent is used in an amount from 0.05 to 0.25 part by weight per part by weight of substituted malonic ester III.

4. A process according to any one of claims 1 to 3, characterized in that an alkyl acetate, an unhalogenated or halogenated aliphatic or aromatic hydrocarbon a long-chain aliphatic alcohol, a long-chain aliphatic nitrile, a water-insoluble ether or a mixture of these solvents is used as solvent.

5. A process according to any one of claims 1 to 4, characterized in that it is carried out batchwise and, after the hydrogenation, the dehydrating agent is separated off together with the hydrogenation catalyst by means of a conventional solid-liquid separation.

6. A process according to claim 5, characterized in that the dehydrating agent is freed of water in the presence of the hydrogenation catalyst together with which it was separated off and is then used together with the hydrogenation catalyst for a new batch.

7. A process according to claim 5, characterized in that the dehydrating agent separated off together with the hydrogenation catalyst is dissolved in water and the residual hydrogenation catalyst is used for a new batch.

8. A process according to any one of claims 1 to 4, characterized in that the hydrogenation is carried out continuously on a fixed bed which is of layered construction if desired and is made up of catalyst and dehydrating agent, over which the substituted malonic ester III trickles while the hydrogen is passed through in co-current or counter-current.

9. A process according to claim 8, characterized in that if desired the fixed bed is washed with a solvent to remove organic impurities, absorbed water is then removed from the dehydrating agent in the fixed bed, and the latter is reused for the hydrogenation.

## Revendications

1. Procédé de fabrication d'esters de l'acide aminomalonique de formule générale I : 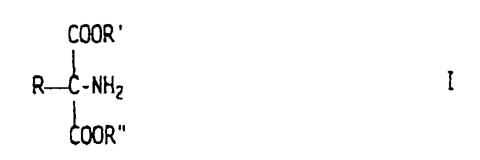 dans laquelle R' et R" peuvent être identiques ou différents et représentent des radicaux alkyle, cycloalkyle, aryle, alcaryle ou aralkyle, R a la même signification que R' et R" et peut en outre représenter un hydrogène, ainsi que le cas échéant de leurs sels de formule générale II : 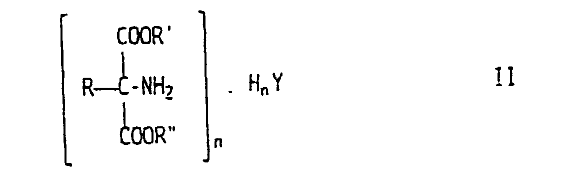 dans laquelle R', R" et R ont la signification donnée pour la formule I. Y représente un anion acide et n représente la basicité de l'acide HₙY par hydrogénation catalytique d'un ester malonique substitué de formule générale III : 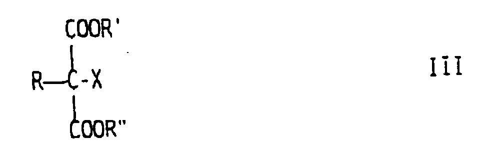 dans laquelle R', R" et R ont la signification indiquée et X représente un groupe nitro, un groupe nitroso ou, lorsque R représente un hydrogène, un groupe imino que l'on peut obtenir par une transposition à partir du groupe nitroso, et qui peut présenter une substitution d'O,
dans un solvant inerte et le cas échéant par addition d'un acide HₙY au mélange d'hydrogénation,
caractérisé en ce qu'
on conduit l'hydrogénation en présence d'un agent desséchant ainsi que d'un solvant ou mélange de solvants dans lequel le sel d'ester aminomalonique II est largement insoluble.

2. Procédé selon la revendication 1,
caractérisé en ce qu'
on utilise comme dessicateur le chlorure de calcium, le sulfate de sodium, le sulfate de magnésium, l'acétate de sodium, le carbonate de sodium, le carbonate de potassium, l'oxyde d'aluminium, un gel de silice, le sulfate de calcium, un échangeur d'ions à base de résine synthétique sèche, un tamis moléculaire ou un mélange de ces matières en une quantité de 0,02 à 4,0 partie en poids par partie en poids d'ester malonique substitué III.

3. Procédé selon la revendication 2,
caractérisé en ce qu'
on utilise le dessicateur en une quantité de 0,05 à 0,25 partie en poids par partie en poids d'ester malonique substitué III.

4. Procédé selon l'une des revendications 1 à 3,
caractérisé en ce qu'
on utilise comme solvant un acétate d'alkyle, un hydrocarbure aliphatique ou aromatique éventuellement halogéné, un alcool aliphatique à longue chaîne, un nitrile aliphatique à longue chaîne, un éther insoluble dans l'eau ou un mélange de ces solvants.

5. Procédé selon l'une des revendications 1 à 4,
caractérisé en ce qu'
• on travaille par étapes, et
• après l'hydrogénation on sépare le dessicateur avec le catalyseur d'hydrogénation par une séparation solide-liquide habituelle.

6. Procédé selon la revendication 5,
caractérisé en ce qu'
• on débarrasse de l'eau le dessicateur en présence du catalyseur d'hydrogénation séparé avec lui, puis
• on l'utilise avec le catalyseur d'hydrogénation pour une nouvelle opération.

7. Procédé selon la revendication 5,
caractérisé en ce qu'
• on dissout dans l'eau le dessicateur séparé avec du catalyseur d'hydrogénation, et
• on utilise le catalyseur d'hydrogénation restant pour une nouvelle opération.

8. Procédé selon l'une des revendications 1 à 4,
caractérisé en ce qu'
on conduit l'hydrogénation de façon continue sur un lit fixe de catalyseur et du dessicateur constitué éventuellement par couches, sur lequel l'ester malonique substitué III coule, tandis qu'on fait passer l'hydrogène dans un courant de même sens ou de sens opposé.

9. Procédé selon la revendication 8,
caractérisé en ce qu'
• on débarrasse le dessicateur de l'eau qu'il a absorbée dans le lit fixe, le cas échéant après lavage du lit fixe avec un solvant pour enlever les impuretés organiques, et
• on utilise à nouveau le lit fixe pour l'hydrogénation.
